# EUROPEAN PATENT APPLICATION

(11) **EP 4 537 827 A1**
(43) Date of publication of application: **16.04.2025**
(21) Application number: 23830288.9
(22) Date of filing: 28.06.2023
(51) Int. Cl.: A61K 31/53, A61K 31/706, A61K 31/7052, A61K 31/519, A61K 31/5377, C07D 487/04, C07H 19/23, C07F 9/6561, A61P 31/14, A61P 1/02, A61P 29/00, A61P 31/20, A61P 31/12, A61P 31/16, A61P 11/00

(54) **METHOD FOR TREATING FELINE CORONAVIRUS OR CALICIVIRUS INFECTION**

(30) Priority: 28.06.2022 CN 202210744698; 09.01.2023 CN 202310030217
(71) Applicant: Vigonvita Life Sciences Co., Ltd., Suzhou, Jiangsu 215123 (CN); Shanghai Institute of Materia Medica, Chinese Academy of Sciences, Shanghai 201203 (CN); Wuhan Institute Of Virology, Chinese Academy of Sciences, Wuhan, Hubei 430071 (CN)
(72) Inventor: TIAN, Guanghui, Suzhou, Jiangsu 215123 (CN); XIE, Yuanchao, Suzhou, Jiangsu 215123 (CN); HE, Yang, Suzhou, Jiangsu 215123 (CN); YANG, Rulei, Suzhou, Jiangsu 215123 (CN); CHENG, Yong, Suzhou, Jiangsu 215123 (CN)
(74) Representative: karo IP
(86) International application number: PCT/CN2023/102984
(87) International publication number: WO 2024/002112

(57) **Abstract**

The present disclosure relates to a compound represented by formula (I) for use in resisting feline coronavirus or calicivirus infection. The compound represented by formula (I) efficiently inhibits the replication of feline coronavirus or calicivirus, and has low toxic and side effects, high oral bioavailability, and good druggability, thus being useful for the treatment of diseases caused by feline coronavirus or calicivirus infection.

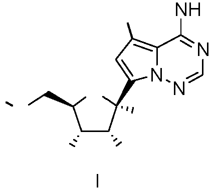

## Description

The present disclosure claims priority to the previously filed Chinese patent application Nos. 202310030217.6 and 202210744698.2, the contents and disclosures of which are incorporated herein by reference in their entirety.

### TECHNICAL FIELD

The present disclosure relates to the field of veterinary medicine, and particularly to a nucleoside compound and a combination formulation thereof for use in the treatment of feline coronavirus or calicivirus infection.

### BACKGROUND

Feline coronavirus (FCoV) belongs to the Coronaviridae family, which is a group of enveloped positive-strand RNA viruses commonly found in cats. In nature, FCoV exists in two distinct biotypes: feline enteric coronavirus (FECV) and feline infectious peritonitis virus (FIPV), with the latter being a mutated form of FECV. FECV infection is widespread among cats, and it is estimated that 40%-80% of cats worldwide carry the virus. FECV chronically infects the gastrointestinal epithelial cells of cats and is usually transmitted via the fecal-oral route. FECV infections in cats are mostly asymptomatic, with some cats experiencing diarrhea, vomiting, loss of appetite, and fever. The FIPV biotype emerges after single nucleotide polymorphisms or deletions (which inactivate the viral 3c protease gene in FECV), and also involves mutations in the viral spike protein. The inactivation of the 3c protease alters the cellular tropism, enabling the virus to replicate within macrophages, and thus promoting the systemic spread of FIPV and the onset of feline infectious peritonitis (FIP). FIP is a progressive immune-related disease in cats. The FIP disease can be classified into "wet" and "dry" forms of FIP. Wet FIP is associated with inflammation of the visceral serosa and omentum, resulting in exudation of fluid into the abdomen and/or thorax. Dry FIP is characterized by granulomatous involvement of parenchymatous organs such as the liver, central nervous system, eyes, and the like. The development of either the wet or dry form of FIP is always fatal. FIP is a major problem in environments with high cat density (e.g., multi-cat households, catteries, shelters, and cat rescue facilities). The disease is most prevalent in younger cats (under 3 years old), particularly kittens, because the higher levels of FECV replication increase the likelihood of mutations to the FIPV biotype and decrease resistance to viruses carrying these mutations. FIP is the leading cause of death in cats under 2 years old, with an estimated global mortality rate of 0.3% to 1% in cats.

Feline calicivirus is a highly contagious, non-enveloped, single-stranded RNA virus with a spherical capsid. It can cause moderate, self-limiting, acute oral problems and upper respiratory tract diseases and is one of the most common pathogens for feline infectious upper respiratory tract diseases. At present, no targeted medicaments are available for the feline stomatitis problem caused by feline calicivirus. Supportive therapies with hormones or antibiotics are generally adopted, but the condition is prone to recurrence. In severe cases, full-mouth tooth extraction surgery is performed, which causes great pain to the cat.

Currently, there is no approved vaccine or effective antiviral therapy for treating FIP or feline stomatitis. Therefore, there is a great need to develop effective medicaments for treating diseases related to feline coronavirus or calicivirus infection, especially oral medicaments that are convenient to administer.

### SUMMARY

### Problems to be solved by the present disclosure

In order to solve the above problems, the present disclosure provides a medicament for treating diseases related to feline coronavirus or calicivirus infection. With significant anti-feline coronavirus (FIPV) activity and high oral bioavailability, this medicament can be used for preventing and treating feline infectious peritonitis and feline stomatitis.

### Solutions to the problems

The present disclosure provides a compound represented by formula I, or a stereoisomer, a solvate, a hydrate, a prodrug, a stable isotopic derivative and a pharmaceutically acceptable salt thereof for use in the preparation of an inhibitor for inhibiting replication of feline coronavirus or calicivirus; and/or a compound represented by formula I, or a stereoisomer, a solvate, a hydrate, a prodrug, a stable isotopic derivative and a pharmaceutically acceptable salt thereof for use in the preparation of a medicament for treating and/or preventing and alleviating a related disease caused by feline coronavirus or calicivirus infection: 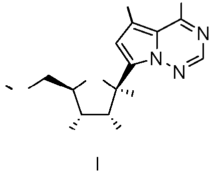 wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 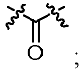
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

The present disclosure provides a composition comprising a compound represented by formula I, or a stereoisomer, a solvate, a hydrate, a prodrug, a stable isotopic derivative and a pharmaceutically acceptable salt thereof for use in the preparation of an inhibitor for inhibiting replication of feline coronavirus or calicivirus;
and/or a composition comprising a compound represented by formula I, or a stereoisomer, a solvate, a hydrate, a prodrug, a stable isotopic derivative and a pharmaceutically acceptable salt thereof for use in the preparation of a medicament for treating and/or preventing and alleviating a related disease caused by feline coronavirus or calicivirus infection: 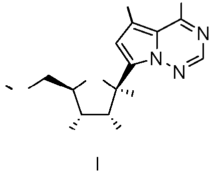
wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 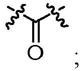
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

### Effects of the present disclosure

(a) The compound of formula I described herein can effectively inhibit the replication of feline coronavirus or calicivirus, and has little toxic effect on normal feline cells.
(b) The compound of formula I described herein has high *in-vivo* inhibitory activity against feline infectious peritonitis viruses.
(c) The compound of formula I described herein has low toxic and side effects in cats, high oral bioavailability, metabolic stability, and good druggability. This suggests that the active compound of the present disclosure has good pharmaceutical prospects in the field of treatment of feline coronavirus or calicivirus infection.

### DETAILED DESCRIPTION

In order to make the technical solutions and beneficial effects of the present disclosure more comprehensible, detailed description is provided below by way of specific examples. Unless defined otherwise, the technical and scientific terms used herein have the same meanings as those in the technical field to which the present disclosure relates.

In one aspect, the present disclosure provides a compound represented by formula I, or a stereoisomer, a solvate, a hydrate, a prodrug, a stable isotopic derivative and a pharmaceutically acceptable salt thereof for use in the preparation of an inhibitor for inhibiting replication of feline coronavirus or calicivirus: 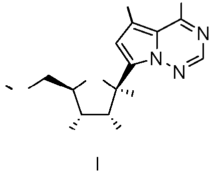 wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 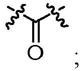
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

In certain embodiments, R₁ in formula I is selected from hydrogen, and substituted or unsubstituted C₁₋₁₈ alkanoyl and C₃₋₇ cycloalkylformyl.

In certain embodiments, R₁ in formula I is selected from hydrogen, and substituted or unsubstituted C₁₋₁₈ alkanoyl and C₃₋₆ cycloalkylformyl.

In certain embodiments, R₁ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₁ is selected from hydrogen, amino, hydroxy, and halogen.

In certain embodiments, Q₁ is selected from hydrogen, amino, and halogen.

In certain embodiments, Q₁ is selected from hydrogen and amino.

In certain embodiments, R₂ in formula I is selected from hydrogen, and substituted or unsubstituted C₁₋₁₈ alkanoyl and C₃₋₇ cycloalkylformyl.

In certain embodiments, R₂ in formula I is selected from hydrogen, and substituted or unsubstituted C₁₋₁₈ alkanoyl and C₃₋₆ cycloalkylformyl.

In certain embodiments, R₂ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₂ is selected from hydrogen, amino, hydroxy, and halogen.

In certain embodiments, Q₂ is selected from hydrogen, amino, and halogen.

In certain embodiments, Q₂ is selected from hydrogen and amino.

In certain embodiments, R₁ and R₂ in formula I are not simultaneously hydrogen.

In certain embodiments, R₁ and R₂ in formula I are connected with each other to form

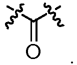

In certain embodiments, R₃ in formula I is selected from hydrogen, and substituted or unsubstituted C₁₋₁₈ alkanoyl and C₃₋₇ cycloalkylformyl.

In certain embodiments, R₃ in formula I is selected from hydrogen, and substituted or unsubstituted C₁₋₁₈ alkanoyl and C₃₋₆ cycloalkylformyl.

In certain embodiments, R₃ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₃ is selected from hydrogen, amino, hydroxy, and halogen.

In certain embodiments, Q₃ is selected from hydrogen, amino, and halogen.

In certain embodiments, Q₃ is selected from hydrogen and amino.

In certain embodiments, R₄ in formula I is selected from hydrogen, deuterium, and halogen.

In certain embodiments, R₄ in formula I is selected from hydrogen, deuterium, fluorine, chlorine, and iodine.

In certain embodiments, the compound represented by formula I is selected from any one of the following:

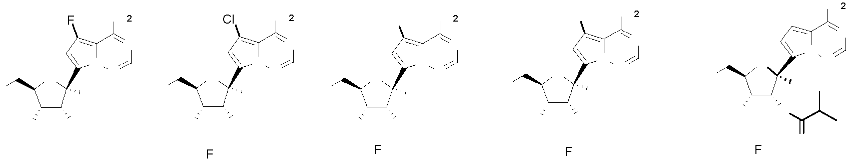

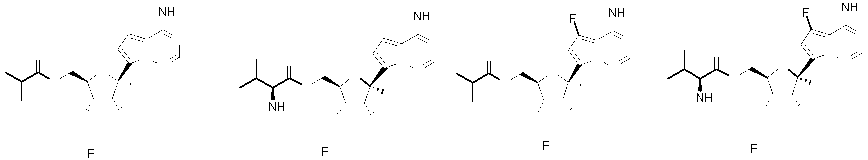

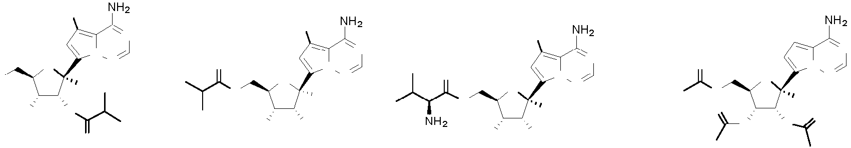

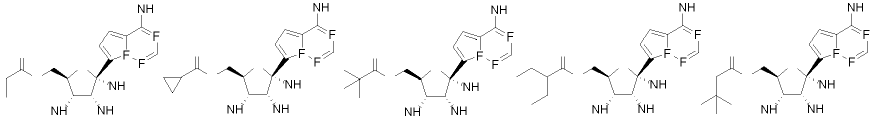

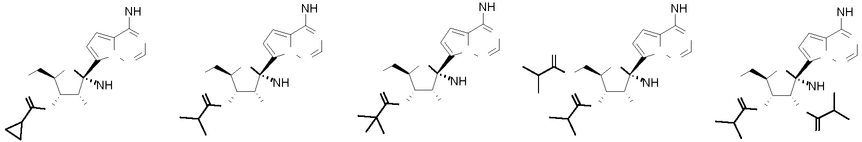

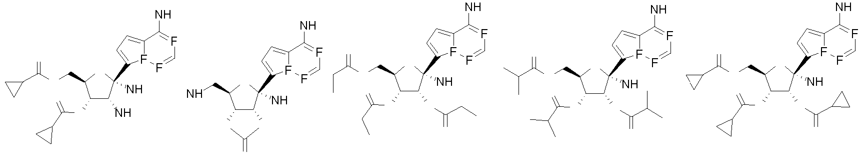

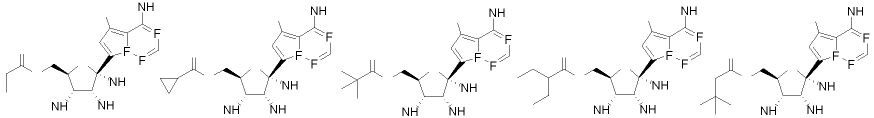

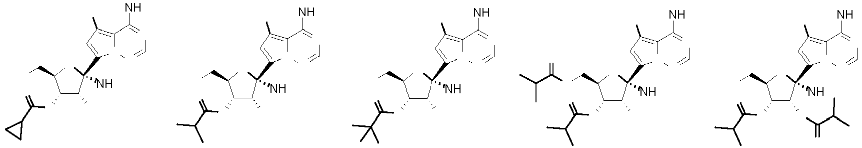

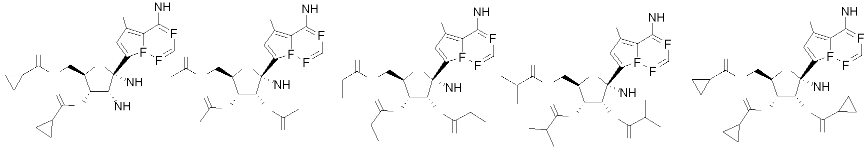

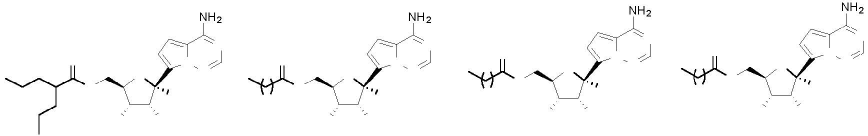

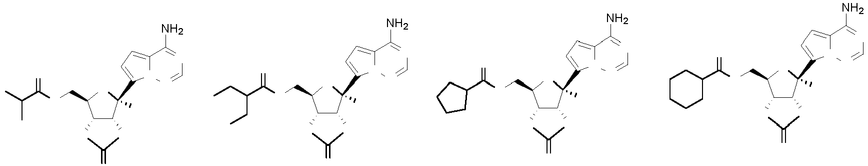

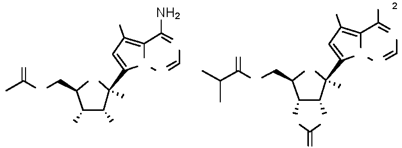

In another aspect, the present disclosure provides a compound represented by formula I, or a stereoisomer, a solvate, a hydrate, a prodrug, a stable isotopic derivative and a pharmaceutically acceptable salt thereof for use in the preparation of a medicament for treating and/or preventing and alleviating a related disease caused by feline coronavirus or calicivirus infection: 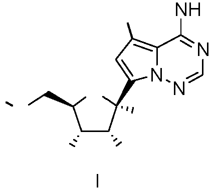 wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 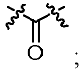
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

In certain embodiments, R₁ in formula I is selected from hydrogen, and substituted or unsubstituted C₁₋₁₈ alkanoyl and C₃₋₇ cycloalkylformyl.

In certain embodiments, R₁ in formula I is selected from hydrogen, and substituted or unsubstituted C₁₋₁₈ alkanoyl and C₃₋₆ cycloalkylformyl.

In certain embodiments, R₁ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₁ is selected from hydrogen, amino, hydroxy, and halogen.

In certain embodiments, Q₁ is selected from hydrogen, amino, and halogen.

In certain embodiments, Q₁ is selected from hydrogen and amino.

In certain embodiments, R₂ in formula I is selected from hydrogen, and substituted or unsubstituted C₁₋₁₈ alkanoyl and C₃₋₇ cycloalkylformyl.

In certain embodiments, R₂ in formula I is selected from hydrogen, and substituted or unsubstituted C₁₋₁₈ alkanoyl and C₃₋₆ cycloalkylformyl.

In certain embodiments, R₂ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₂ is selected from hydrogen, amino, hydroxy, and halogen.

In certain embodiments, Q₂ is selected from hydrogen, amino, and halogen.

In certain embodiments, Q₂ is selected from hydrogen and amino.

In certain embodiments, R₁ and R₂ in formula I are not simultaneously hydrogen.

In certain embodiments, R₁ and R₂ in formula I are connected with each other to form

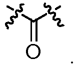

In certain embodiments, R₃ in formula I is selected from hydrogen, and substituted or unsubstituted C₁₋₁₈ alkanoyl and C₃₋₇ cycloalkylformyl.

In certain embodiments, R₃ in formula I is selected from hydrogen, and substituted or unsubstituted C₁₋₁₈ alkanoyl and C₃₋₆ cycloalkylformyl.

In certain embodiments, R₃ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₃ is selected from hydrogen, amino, hydroxy, and halogen.

In certain embodiments, Q₃ is selected from hydrogen, amino, and halogen.

In certain embodiments, Q₃ is selected from hydrogen and amino.

In certain embodiments, R₄ in formula I is selected from hydrogen, deuterium, and halogen.

In certain embodiments, R₄ in formula I is selected from hydrogen, deuterium, fluorine, chlorine, and iodine.

In certain embodiments, the compound represented by formula I is selected from any one of the following:

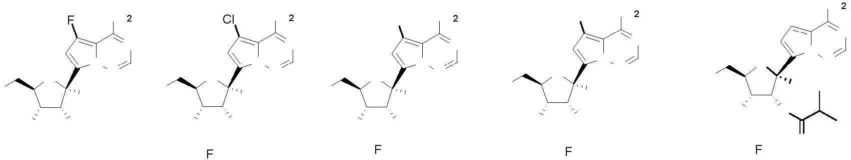

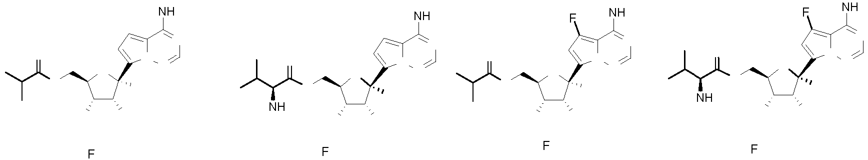

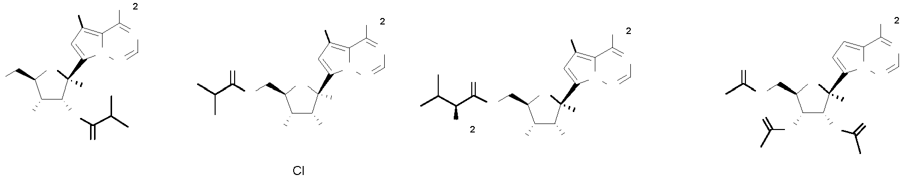

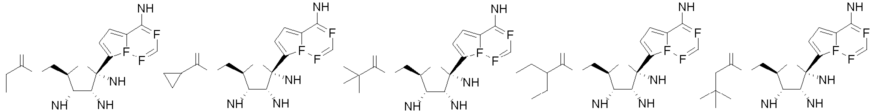

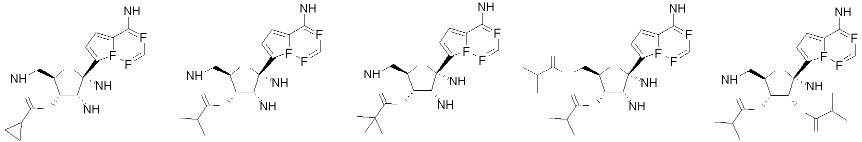

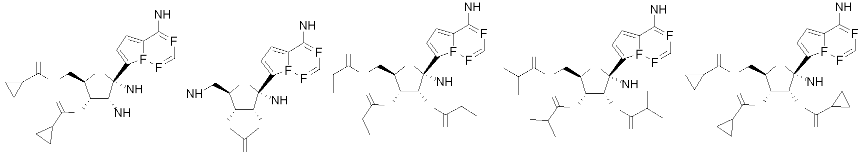

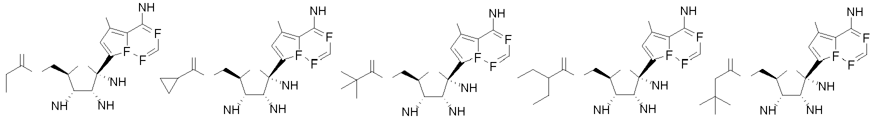

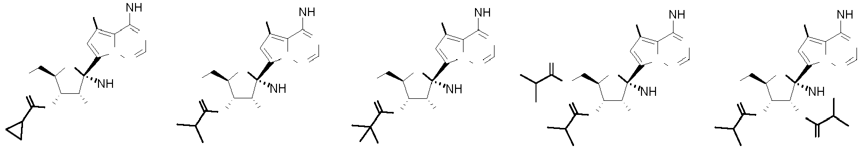

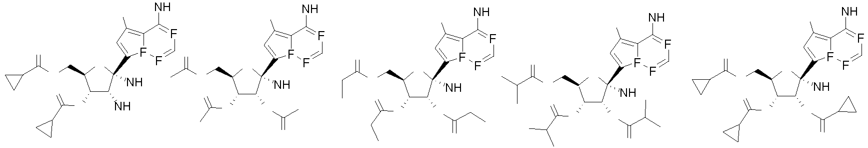

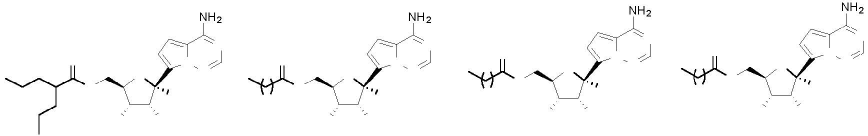

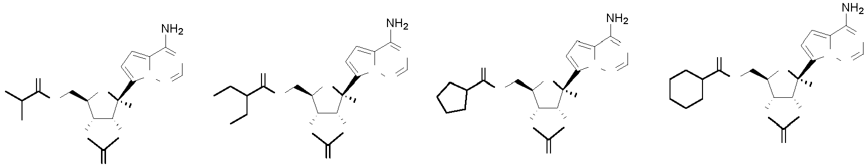

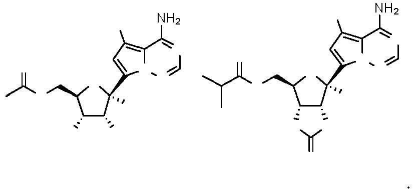

In certain embodiments, the related disease caused by feline coronavirus or calicivirus infection is selected from feline infectious peritonitis and feline stomatitis.

In another aspect, the present disclosure provides a composition comprising a compound represented by formula I, or a stereoisomer, a solvate, a hydrate, a prodrug, a stable isotopic derivative and a pharmaceutically acceptable salt thereof for use in the preparation of an inhibitor for inhibiting replication of feline coronavirus or calicivirus: 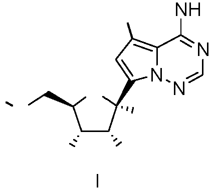 wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 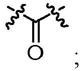
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

In certain embodiments, R₁ in formula I is selected from hydrogen, and substituted or unsubstituted C₁₋₁₈ alkanoyl and C₃₋₇ cycloalkylformyl.

In certain embodiments, R₁ in formula I is selected from hydrogen, and substituted or unsubstituted C₁₋₁₈ alkanoyl and C₃₋₆ cycloalkylformyl.

In certain embodiments, R₁ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₁ is selected from hydrogen, amino, hydroxy, and halogen.

In certain embodiments, Q₁ is selected from hydrogen, amino, and halogen.

In certain embodiments, Q₁ is selected from hydrogen and amino.

In certain embodiments, R₂ in formula I is selected from hydrogen, and substituted or unsubstituted C₁₋₁₈ alkanoyl and C₃₋₇ cycloalkylformyl.

In certain embodiments, R₂ in formula I is selected from hydrogen, and substituted or unsubstituted C₁₋₁₈ alkanoyl and C₃₋₆ cycloalkylformyl.

In certain embodiments, R₂ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₂ is selected from hydrogen, amino, hydroxy, and halogen.

In certain embodiments, Q₂ is selected from hydrogen, amino, and halogen.

In certain embodiments, Q₂ is selected from hydrogen and amino.

In certain embodiments, R₁ and R₂ in formula I are not simultaneously hydrogen.

In certain embodiments, R₁ and R₂ in formula I are connected with each other to form

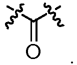

In certain embodiments, R₃ in formula I is selected from hydrogen, and substituted or unsubstituted C₁₋₁₈ alkanoyl and C₃₋₇ cycloalkylformyl.

In certain embodiments, R₃ in formula I is selected from hydrogen, and substituted or unsubstituted C₁₋₁₈ alkanoyl and C₃₋₆ cycloalkylformyl.

In certain embodiments, R₃ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₃ is selected from hydrogen, amino, hydroxy, and halogen.

In certain embodiments, Q₃ is selected from hydrogen, amino, and halogen.

In certain embodiments, Q₃ is selected from hydrogen and amino.

In certain embodiments, R₄ in formula I is selected from hydrogen, deuterium, and halogen.

In certain embodiments, R₄ in formula I is selected from hydrogen, deuterium, fluorine, chlorine, and iodine.

In certain embodiments, the compound represented by formula I is selected from any one of the following:

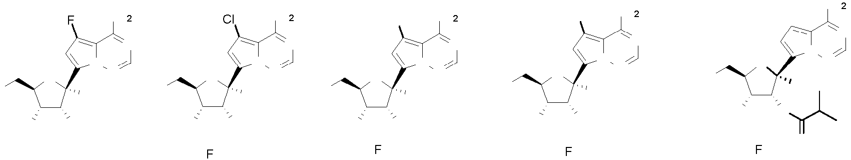

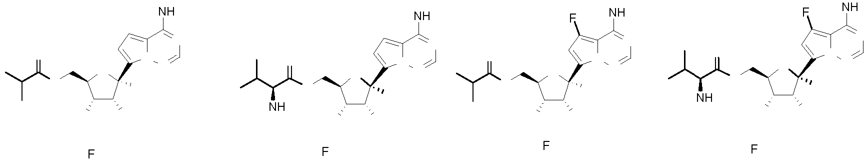

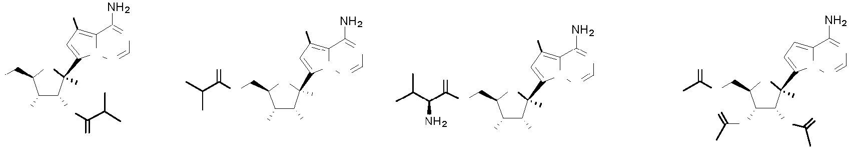

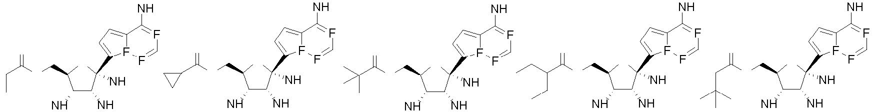

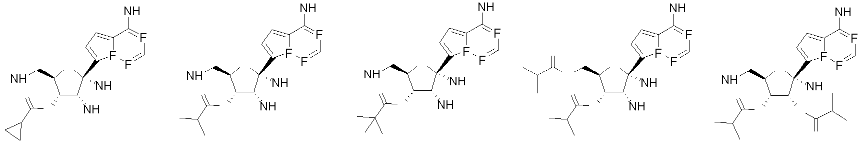

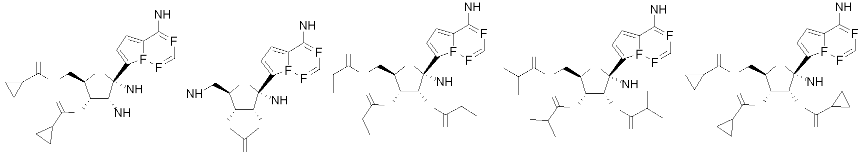

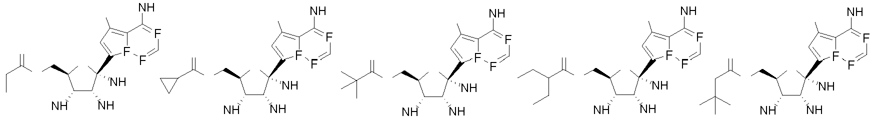

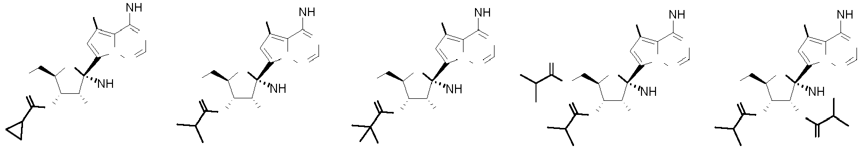

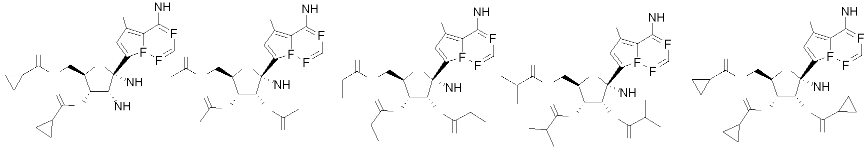

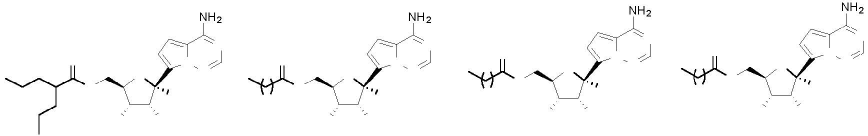

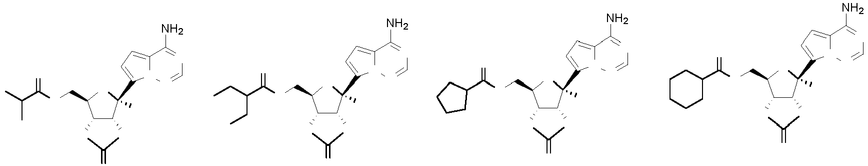

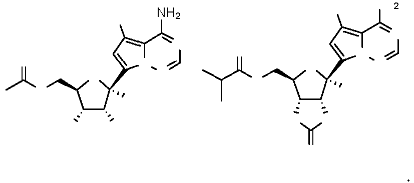

In another aspect, the present disclosure provides a composition comprising a compound represented by formula I, or a stereoisomer, a solvate, a hydrate, a prodrug, a stable isotopic derivative and a pharmaceutically acceptable salt thereof for use in the preparation of a medicament for treating and/or preventing and alleviating a related disease caused by feline coronavirus or calicivirus infection: 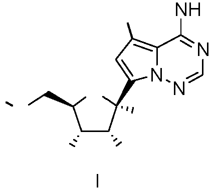 wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 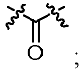
R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

In certain embodiments, R₁ in formula I is selected from hydrogen, and substituted or unsubstituted C₁₋₁₈ alkanoyl and C₃₋₇ cycloalkylformyl.

In certain embodiments, R₁ in formula I is selected from hydrogen, and substituted or unsubstituted C₁₋₁₈ alkanoyl and C₃₋₆ cycloalkylformyl.

In certain embodiments, R₁ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₁ is selected from hydrogen, amino, hydroxy, and halogen.

In certain embodiments, Q₁ is selected from hydrogen, amino, and halogen.

In certain embodiments, Q₁ is selected from hydrogen and amino.

In certain embodiments, R₂ in formula I is selected from hydrogen, and substituted or unsubstituted C₁₋₁₈ alkanoyl and C₃₋₇ cycloalkylformyl.

In certain embodiments, R₂ in formula I is selected from hydrogen, and substituted or unsubstituted C₁₋₁₈ alkanoyl and C₃₋₆ cycloalkylformyl.

In certain embodiments, R₂ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₂ is selected from hydrogen, amino, hydroxy, and halogen.

In certain embodiments, Q₂ is selected from hydrogen, amino, and halogen.

In certain embodiments, Q₂ is selected from hydrogen and amino.

In certain embodiments, R₁ and R₂ in formula I are not simultaneously hydrogen.

In certain embodiments, R₁ and R₂ in formula I are connected with each other to form

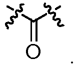

In certain embodiments, R₃ in formula I is selected from hydrogen, and substituted or unsubstituted C₁₋₁₈ alkanoyl and C₃₋₇ cycloalkylformyl.

In certain embodiments, R₃ in formula I is selected from hydrogen, and substituted or unsubstituted C₁₋₁₈ alkanoyl and C₃₋₆ cycloalkylformyl.

In certain embodiments, R₃ in formula I is selected from hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl.

In certain embodiments, Q₃ is selected from hydrogen, amino, hydroxy, and halogen.

In certain embodiments, Q₃ is selected from hydrogen, amino, and halogen.

In certain embodiments, Q₃ is selected from hydrogen and amino.

In certain embodiments, R₄ in formula I is selected from hydrogen, deuterium, and halogen.

In certain embodiments, R₄ in formula I is selected from hydrogen, deuterium, fluorine, chlorine, and iodine.

In certain embodiments, the compound represented by formula I is selected from any one of the following:

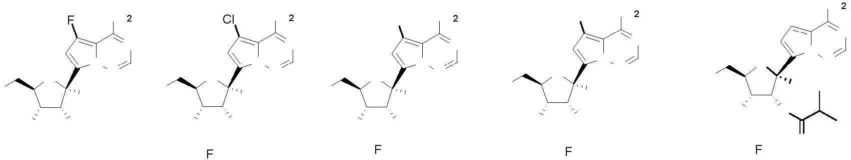

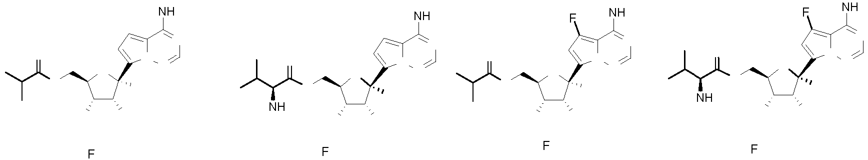

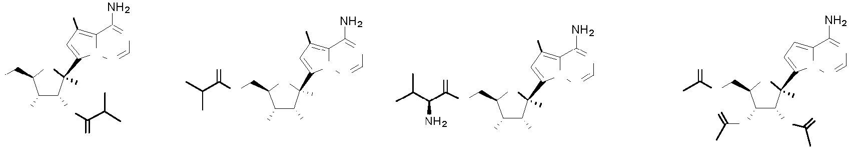

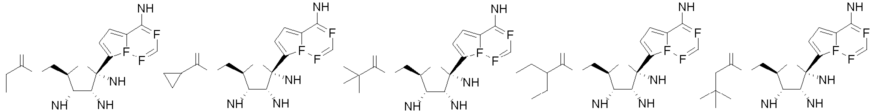

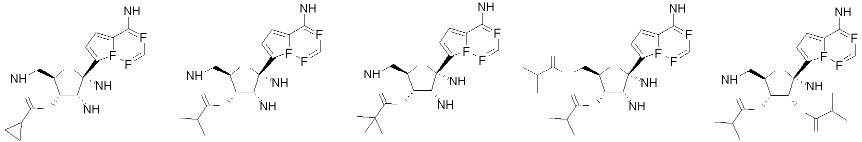

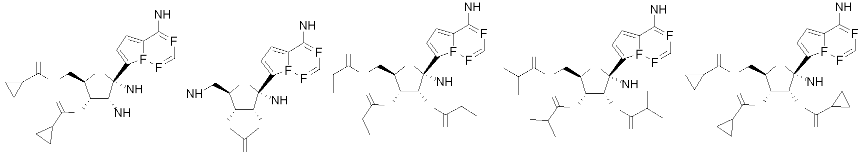

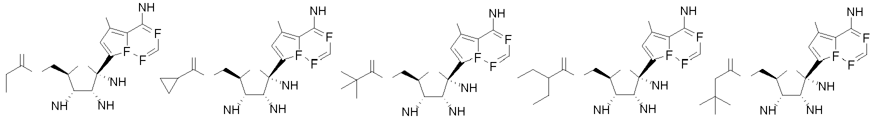

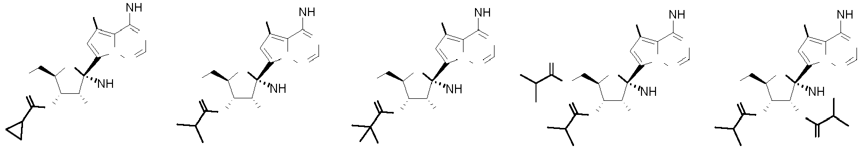

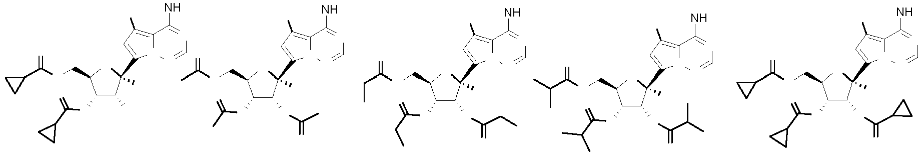

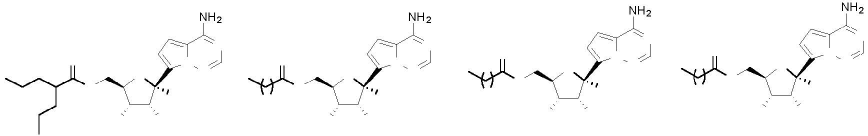

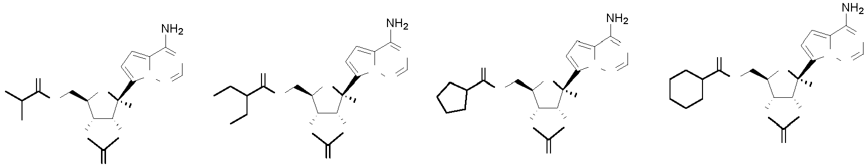

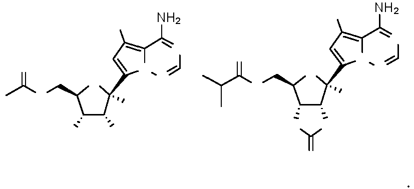

In certain embodiments, the related disease caused by feline coronavirus or calicivirus infection is selected from feline infectious peritonitis and feline stomatitis.

In certain embodiments, the pharmaceutical composition comprises at least one of the compound or the stereoisomer, the solvate, the hydrate, the prodrug, the stable isotopic derivative and the pharmaceutically acceptable salt thereof described above, as well as a pharmaceutically acceptable carrier, diluent or excipient.

In certain embodiments, the pharmaceutical composition further comprises an additional antiviral drug selected from the group consisting of: PF-07321332 (nirmatrelvir), S-217622 (ensitrelvir), FHPI (4-[4-(4-fluorophenyl)-5-pyridin-4-yl-1,3-dihydro-imidazol-2-ylidene]cyclohexa-2,5-dien-1-one), aloxistatin, conivaptan, favipiravir, galidesivir, NHC (EIDD-1931), EIDD-2801, GC-376, lopinavir, ritonavir, nelfinavir, chloroquine, hydroxychloroquine, cyclosporine, carrimycin, baicalin, baicalein, forsythoside, chlorogenic acid, emodin, mycophenolic acid, mycophenolate mofetil, naphthoquine, ciclesonide, ribavirin, penciclovir, leflunomide, teriflunomide, nafamostat, nitazoxanide, darunavir, arbidol, camostat, niclosamide, baricitinib, ruxolitinib, dasatinib, saquinavir, beclabuvir, simeprevir, palivizumab, motavizumab, RSV-IGIV MEDI557, A-60444 (RSV-604), MDT-637, BMS-433771, and pharmaceutically acceptable salts thereof, and combinations thereof.

In certain embodiments, the pharmaceutical composition further comprises an additional antiviral drug selected from the group consisting of: PF-07321332 (nirmatrelvir), S-217622 (ensitrelvir), FHPI (4-[4-(4-fluorophenyl)-5-pyridin-4-yl-1,3-dihydro-imidazol-2-ylidene]cyclohexa-2,5-dien-1-one), aloxistatin, conivaptan, favipiravir, NHC (EIDD-1931), EIDD-2801, baicalin, baicalein, forsythoside, chlorogenic acid, emodin, glycyrrhizic acid, chloroquine, hydroxychloroquine, and nelfinavir.

In certain embodiments, the pharmaceutical composition further comprises an additional antiviral drug selected from the group consisting of: interferons, RNA-dependent RNA polymerase inhibitors (e.g., favipiravir, galidesivir, NHC (EIDD-1931), and EIDD-2801), 3CL protease inhibitors (e.g., GC-376), lopinavir, ritonavir, nelfinavir, S-217622 (ensitrelvir), PF-07321332 (nirmatrelvir), chloroquine, hydroxychloroquine, cyclosporine, carrimycin, baicalin, baicalein, forsythoside, chlorogenic acid, emodin, mycophenolic acid, mycophenolate mofetil, naphthoquine, ciclesonide, ribavirin, penciclovir, leflunomide, teriflunomide, nafamostat, nitazoxanide, darunavir, arbidol, camostat, niclosamide, baricitinib, ruxolitinib, dasatinib, saquinavir, beclabuvir, simeprevir, palivizumab, motavizumab, RSV-IGIVMEDI-557, A-60444(RSV-604), MDT637, BMS-433771, FHPI (4-[4-(4-fluorophenyl)-5-pyridin-4-yl-1,3-dihydro-imidazol-2-ylidene]cyclohexa-2,5-dien-1-one), aloxistatin, conivaptan, and pharmaceutically acceptable salts thereof, and combinations thereof.

In certain embodiments, the pharmaceutical composition further comprises an additional drug selected from the group consisting of: zinc, fingolimod, vitamin C, olmesartan medoxomil, valsartan, losartan, thalidomide, glycyrrhizic acid, artemisinin, dihydroartemisinin, artesunate, artemisone, azithromycin, escin, naproxen, and combinations thereof.

In certain embodiments, the pharmaceutical composition further comprises an additional drug selected from the group consisting of: (Y1) RNA replicase inhibitors (e.g., favipiravir, galidesivir, NHC, and EIDD-2801); (Y2) lopinavir; (Y3) ritonavir; (Y4) chloroquine, hydroxychloroquine, or a pharmaceutically acceptable salt thereof (e.g., chloroquine phosphate), (Y5) nelfinavir; and (Y6) any combination of the above Y1 to Y5.

In certain embodiments, the pharmaceutical composition further comprises an additional drug selected from the group consisting of: interferons, RNA-dependent RNA polymerase inhibitors (e.g., favipiravir, galidesivir, NHC, and EIDD-2801), 3CL protease inhibitors (e.g., GC-376), lopinavir, ritonavir, nelfinavir, chloroquine, hydroxychloroquine, cyclosporine, carrimycin, baicalin, baicalein, forsythoside, chlorogenic acid, emodin, mycophenolic acid, mycophenolate mofetil, naphthoquine, ciclesonide, ribavirin, penciclovir, leflunomide, teriflunomide, nafamostat, nitazoxanide, darunavir, arbidol, camostat, niclosamide, baricitinib, ruxolitinib, dasatinib, saquinavir, beclabuvir, simeprevir, palivizumab, motavizumab, RSV-IGIVMEDI557, A-60444 (RSV-604), MDT-637, BMS-433771, and pharmaceutically acceptable salts thereof, and combinations thereof. The interferon includes one or more of interferon α-2a, interferon α-2b, interferon α-n1, interferon α-n3, interferon β-1a, and interferon β-1b.

In certain embodiments, the pharmaceutical composition further comprises an additional drug selected from the group consisting of: for example, the use of "anti-inflammatory signal transduction modulators" (referred to herein as AISTM), such as phosphodiesterase inhibitors (e.g., PDE-4, PDE-5 or PDE-7 specific), transcription factor inhibitors (e.g., blocking NFκB by IKK inhibition), or kinase inhibitors (e.g., blocking P38MAP, JNK, PI3K, EGFR, or Syk), which is a logical approach to interrupt inflammation, because these small molecules target a limited number of common intracellular pathways, i.e., those signal transduction pathways that are key points of intervention for anti-inflammatory therapy (see review by P.J. Barnes, 2006). These non-limiting additional therapeutic agents include: 5-(2,4-difluoro-phenoxy)-1-isobutyl-1H-indazole-6-carboxylic acid (2-dimethylamino-ethyl)-amide (P38Map kinase inhibitor ARRY-797); 3-cyclopropylmethoxy-N-(3,5-dichloro-pyridin-4-yl)-4-difluoromethoxy-benzamide (PDE-4 inhibitor roflumilast); 4-[2-(3-cyclopentyloxy-4-methoxyphenyl)-2-phenyl-ethyl]-pyridine (PDE-4 inhibitor CDP-840); N-(3,5-dichloro-4-pyridinyl)-4-(difluoromethoxy)-8-[(methylsulfonyl)amino]-1-dibenzofurancarboxamide (PDE-4 inhibitor oglemilast); N-(3,5-dichloropyridin-4-yl)-2-[1-(4-fluorobenzyl)-5-hydroxy-1H-indol-3-yl]-2-oxo-acetamide (PDE-4 inhibitor AWD12-281); 8-methoxy-2-trifluoromethyl-quinoline-5-carboxylic acid (3,5-dichloro-1-oxy-pyridin-4-yl)-amide (PDE-4 inhibitor Sch351591); 4-[5-(4-fluorophenyl)-2-(4-methanesulfinyl-phenyl)-1H-imidazol-4-yl]-pyridine (P38 inhibitor SB-203850); 4-[4-(4-fluoro-phenyl)-1-(3-phenyl-propyl)-5-pyridin-4-yl-1H-imidazol-2-yl]-but-3-yn-1-ol (P38 inhibitor RWJ-67657); 4-cyano-4-(3-cyclopentyloxy-4-methoxy-phenyl)-cyclohexanecarboxylic acid 2-diethylamino-ethyl ester (2-diethyl-ethyl ester prodrug of cilomilast, PDE-4 inhibitor); (3-chloro-4-fluorophenyl)-[7-methoxy-6-(3-morpholin-4-yl-propoxy)-quinazolin-4-yl]-amine (gefitinib, EGFR inhibitor); and 4-(4-methyl-piperazin-1-ylmethyl)-N-[4-methyl-3-(4-pyridin-3-yl-pyrimidin-2-ylamino)-phenyl]-benzamide (imatinib, EGFR inhibitor).

In certain embodiments, a unit dose of the pharmaceutical composition is 0.001-1000 mg.

In certain embodiments, the pharmaceutical composition comprises 0.01%-99.99% of the compound described above based on the total weight of the composition. In certain embodiments, the pharmaceutical composition comprises 0.1%-99.9% of the compound described above. In certain embodiments, the pharmaceutical composition comprises 0.5%-99.5% of the compound described above. In certain embodiments, the pharmaceutical composition comprises 1%-99% of the compound described above. In certain embodiments, the pharmaceutical composition comprises 2%-98% of the compound described above.

In certain embodiments, the pharmaceutical composition comprises 0.01%-99.99% of the pharmaceutically acceptable carrier, diluent or excipient based on the total weight of the composition. In certain embodiments, the pharmaceutical composition comprises 0.1%-99.9% of the pharmaceutically acceptable carrier, diluent or excipient. In certain embodiments, the pharmaceutical composition comprises 0.5%-99.5% of the pharmaceutically acceptable carrier, diluent or excipient. In certain embodiments, the pharmaceutical composition comprises 1%-99% of the pharmaceutically acceptable carrier, diluent or excipient. In certain embodiments, the pharmaceutical composition comprises 2%-98% of the pharmaceutically acceptable carrier, diluent or excipient.

All the compounds involved in the present disclosure, as well as mixtures, compositions and the like comprising the compounds of the present disclosure, may be administered to an organism via any route of administration. The route of administration may be oral administration, intravenous injection, intramuscular injection, subcutaneous injection, rectal administration, vaginal administration, sublingual administration, nasal inhalation, oral inhalation, eye drops, or topical or systemic transdermal administration.

All the compounds involved in the present disclosure, as well as mixtures, compositions and the like comprising the compounds of the present disclosure, may be prepared into a single dose, which contains the active compound of the present disclosure along with carriers, excipients, and the like; the dosage forms for administration may be tablets, capsules, injections, granules, powders, suppositories, pills, creams, pastes, gels, pulveres, oral solutions, inhalants, suspensions, dry suspensions, patches, lotions, and the like. Such dosage forms may contain ingredients commonly used in pharmaceutical formulations, such as diluents, absorbents, wetting agents, binders, disintegrants, colorants, pH adjusters, antioxidants, bacteriostatic agents, isotonicity adjusters, anti-adherents, and the like.

Suitable formulas for the various dosage forms described above are available from public sources, such as Remington: The Science and Practice of Pharmacy, 21st edition, published by Lippincott Williams & Wilkins in 2006, and Rowe, Raymond C., Handbook of Pharmaceutical Excipients, published by Pharmaceutical Press, Chicago in 2005. Therefore, these dosage forms can be readily prepared by those skilled in the art.

Based on the nature and severity of the disease, age, sex and body weight of the individual, the route of administration, and other factors, different administration doses can be selected; the compound of the present disclosure may be administered at a daily dose of 0.01 to 500 mg/kg, preferably a daily dose of 1 to 100 mg/kg, and may be administered either as a single dose or in multiple doses.

### Terms and definitions:

Unless otherwise stated, the terms used in the specification and claims have the following meanings. The term "C₁₋₂₀ alkyl" refers to a saturated aliphatic hydrocarbon group, which is a linear or branched group containing 1 to 20 carbon atoms, preferably alkyl containing 1 to 12 (e.g., 1, 2, 3, 4, 5, 6, 7, 8, 9, 10, 11, and 12) carbon atoms, and more preferably alkyl containing 1 to 6 carbon atoms. Non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted. When substituted, it may be substituted at any accessible connection site, where the substituent is preferably one or more substituents independently and optionally selected from a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "C₁₋₂₀ alkanoyl" refers to the group C₁₋₂₀ alkyl-C(O)-, where the "C₁₋₂₀ alkyl" is as defined above.

The term "C₃₋₁₀ cycloalkyl" refers to a saturated or partially unsaturated monocyclic or polycyclic hydrocarbon substituent, and the cycloalkyl ring contains 3 to 10 carbon atoms, preferably 3 to 8 (e.g., 3, 4, 5, 6, 7, and 8) carbon atoms, and more preferably 3 to 6 carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; the polycyclic cycloalkyl includes spiro cycloalkyl, fused cycloalkyl, and bridged cycloalkyl.

The cycloalkyl may be substituted or unsubstituted. When substituted, it may be substituted at any accessible connection site, where the substituent is preferably one or more substituents independently and optionally selected from halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "C₃₋₁₀ cycloalkylformyl" refers to the group C₃₋₁₀ cycloalkyl-C(O)-, where the "C₃₋₁₀ cycloalkyl" is as defined above.

The term "halogen" refers to fluorine, chlorine, bromine, or iodine.

The term "hydroxy" refers to -OH.

The term "amino" refers to -NH₂.

The term "cyano" refers to -CN.

The term "substituted" means that one or more, preferably up to 5, more preferably 1 to 3 hydrogen atoms in the group are independently substituted with a corresponding number of substituents. It goes without saying that a substituent is only present in its possible chemical position, and those skilled in the art will be able to determine (experimentally or theoretically) possible or impossible substitutions without undue effort. For example, it may be unstable when an amino or hydroxy group having free hydrogen is bound to a carbon atom having an unsaturated (e.g., olefinic) bond.

In the chemical structure of the compound described herein, the " 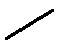 " bond does not specify a configuration; that is, the " 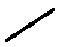 " bond may be " 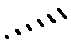 " or " 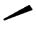 ", or includes both " 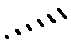 " and " 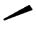 " configurations.

The term "stereoisomer" refers to compounds that have the same chemical structure but differ in the spatial arrangement of atoms or groups. Stereoisomers include enantiomers, diastereomers, conformational isomers (rotamers), geometric (cis/trans) isomers, atropisomers, and the like.

The term "isotopic derivative" refers to compounds that differ in structure only by having one or more enriched isotopic atoms. For example, compounds with the structure of the present disclosure having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such compounds can be used as analytical tools or probes in, for example, biological assays, or may be used as tracers for *in-vivo* diagnostic imaging of diseases, or as tracers for pharmacodynamic, pharmacokinetic or receptor studies. The various deuterated forms of the compounds of the present disclosure mean that each available hydrogen atom connected to a carbon atom may be independently replaced by a deuterium atom. Those skilled in the art can synthesize the deuterated forms of the compounds by reference to the relevant literature. The deuterated forms of the compounds can be prepared using commercially available deuterated starting materials, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain activity comparable to that of non-deuterated compounds, and can achieve better metabolic stability when deuteration occurs at certain specific sites, thereby achieving certain therapeutic advantages.

The term "pharmaceutically acceptable salt" means that the compounds of the present disclosure are present in the form of their pharmaceutically acceptable salts, including acid addition salts and base addition salts. Pharmaceutically acceptable salts are described in pharmaceutical salts described by S. M. Berge in J. Pharmaceutical Sciences (Vol. 66: pp. 1-19, 1977). In the present disclosure, pharmaceutically acceptable non-toxic acid addition salts refer to salts formed by reaction of the compounds of the present disclosure with organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to salts formed by reaction of the compounds of the present disclosure with organic or inorganic bases including, but not limited to, alkali metal salts (e.g., lithium, sodium or potassium salts), alkaline earth metal salts (e.g., calcium or magnesium salts), and organic base salts (e.g., ammonium salts or N⁺(C₁₋₆ alkyl)₄ salts formed by reaction with N group-containing organic bases). The term "solvate" refers to a substance formed by a physical association of the compound of the present disclosure with one or more, preferably 1 to 3, solvent molecules (whether organic or inorganic). The physical association includes hydrogen bonding. In certain cases, for example, when one or more, preferably 1 to 3, solvent molecules are incorporated into the crystal lattice of a crystalline solid, the solvate will be isolated. Exemplary solvates include, but are not limited to, hydrates, ethanolates, methanolates, and isopropanolates. Solvation methods are well known in the art.

The term "prodrug" refers to a substance that can be converted *in vivo* under physiological conditions, such as through hydrolysis in blood, to generate the active prodrug compound.

The term "pharmaceutical composition" refers to a mixture containing one or more of the compounds or the physiologically/pharmaceutically acceptable salts or prodrugs thereof described herein, and other chemical components, as well as other components such as physiologically/pharmaceutically acceptable carriers and excipients. The pharmaceutical composition is intended to promote administration to an organism and facilitate the absorption of the active ingredient so that it can exert its bioactivity.

The terms referred to in the present disclosure are defined above. Those skilled in the art can also interpret the above terms with reference to the prior art. Further description is made below based on the contents of the present disclosure and the definitions of the terms.

The preparation of the compounds and the pharmaceutically acceptable salts thereof described herein is further described below in conjunction with examples, but these examples are not intended to limit the scope of the present disclosure.

Experimental procedures without specific conditions indicated in the examples of the present disclosure are generally conducted according to conventional conditions, or according to conditions recommended by the manufacturers of the starting materials or commercial products. Reagents without specific origins indicated are commercially available conventional reagents.

### Example 1: Preparation of Compound 6

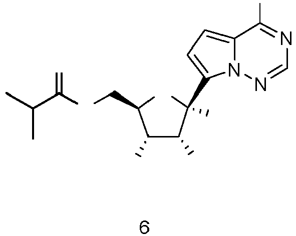

Compound 6 of the present disclosure was prepared using the synthesis method for the related compound A50 described in the specification of WO2021213288A1.

### Example 2: Preparation of Compound 25

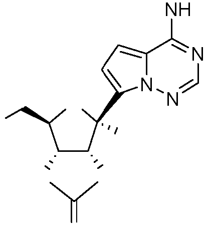

Compound GS-441524 (CAS RN: 1191237-69-0, 85 mg, 0.292 mmol) was added to *N,N-*dimethylformamide, and then carbonyldiimidazole (48 mg, 0.292 mmol) was added. The mixture was stirred at room temperature. After 15 min, water and ethyl acetate were added to the reaction solution. The organic phase was isolated, washed with saturated brine, dried over anhydrous sodium sulfate, and separated by silica gel column chromatography (dichloromethane:methanol = 20: 1) to obtain compound 25 as a white solid (19 mg, yield 21%). ¹ H NMR (500 MHz, DMSO-d6) δ 8.16 (s, 1H), 8.02 (s, 1H), 8.00 (s, 1H), 7.02-6.98 (m, 2H), 5.96 (d, J = 7.9 Hz, 1H), 5.40 (dd, J = 7.8, 4.0 Hz, 1H), 5.28 (t, J = 5.7 Hz, 1H), 4.50 (q, J = 4.7 Hz, 1H), 3.72-3.65 (m, 1H), 3.64-3.58 (m, 1H).

### Example 3: Preparation of Compound 27

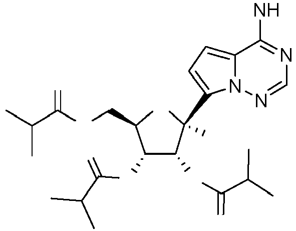

Compound 27 of the present disclosure was prepared using the synthesis method for the related compound A131 described in the specification of WO2021213288A1.

### Example 4: Preparation of Compound 42

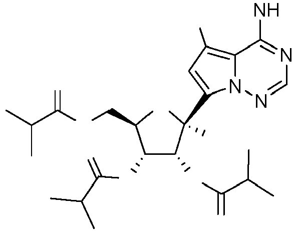

Compound 42 of the present disclosure was prepared using the synthesis method for the related compound A151 described in the specification of WO2021213288A1.

### Example 5: Preparation of Compound 4

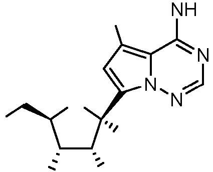

Compound 4 of the present disclosure was prepared using the synthesis method for the related compound A9 described in the specification of WO2021213288A1.

### Example 6: Preparation of Compound 11

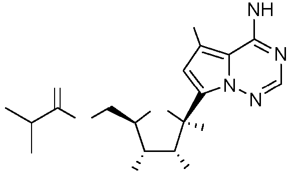

Compound 11 of the present disclosure was prepared using the synthesis method for the related compound A70 described in the specification of WO2021213288A1.

### Example 7: Preparation of Compound 20

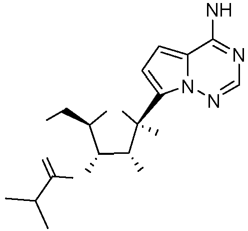

Compound 20 of the present disclosure was prepared using the synthesis method for the related compound A124 described in the specification of WO2021213288A1.

### Example 8: Preparation of Compound 29

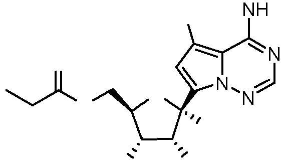

Compound 29 of the present disclosure was prepared using the synthesis method for the related compound A138 described in the specification of WO2021213288A1.

### Example 9: Preparation of Compound 40

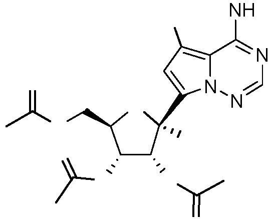

Compound 4 (2 g, 6.84 mmol) and 4-dimethylaminopyridine (83 mg, 0.68 mmol) were dissolved in N,N-dimethylacetamide (20 mL), and then acetic anhydride (2.16 g, 21.20 mmol) was slowly added dropwise under an ice-water bath. The mixture was warmed to room temperature and allowed to react for 1 h. The starting materials were completely reacted as monitored by TLC. Water and ethyl acetate were added to the reaction solution, and the mixture was stirred and left to stand for liquid separation. The organic phase was washed sequentially with 0.1% diluted hydrochloric acid and a saturated sodium bicarbonate solution, then dried over anhydrous sodium sulfate, filtered, concentrated, and purified by column chromatography to obtain compound 40 as a foamy solid (2.16 g, yield 76%). ¹H NMR (400 MHz, DMSO-*d*₆) δ 8.12 - 7.91 (m, 3H), 6.82 (s, 1H), 6.11 (d, *J =* 5.9 Hz, 1H), 5.42 - 5.33 (m, 1H), 4.58 (q, *J* = 4.6 Hz, 1H), 4.39 (dd, *J* = 12.3, 3.3 Hz, 1H), 4.22 (dd, *J* = 12.3, 4.9 Hz, 1H), 2.12 (s, 6H), 2.00 (s, 3H). ESI-MS m/z = 419.2 [M+1]⁺.

### Example 10: Preparation of Compound 52

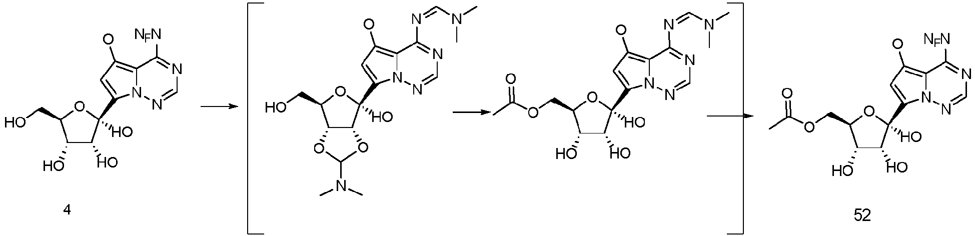

Compound 4 (2 g, 6.84 mmol) was dispersed in tetrahydrofuran (20 mL), and the system was heated to 60 °C. *N*,*N*-Dimethylformamide dimethyl acetal (3.67 g, 30.79 mmol) was slowly added, and the mixture was incubated for reaction for 4 h. The starting materials were completely reacted as detected by TLC. The reaction solution was concentrated to dryness to obtain intermediate 52-1 as an oil. This intermediate was directly used in the next step without separation.

Intermediate 52-1 obtained from the previous step and 4-dimethylaminopyridine (167 mg, 1.37 mmol) were dissolved in dichloromethane (20 mL), and then acetic anhydride (908 mg, 8.89 mmol) was slowly added dropwise under an ice-water bath. The mixture was warmed to room temperature and allowed to react for 1 h. The starting materials were completely reacted as monitored by TLC. Water and ethyl acetate were added to the reaction solution, and the mixture was stirred and left to stand for liquid separation. The aqueous phase was back-extracted with ethyl acetate, and the organic phases were combined, washed sequentially with 0.1% diluted hydrochloric acid and a saturated sodium bicarbonate solution, then dried over anhydrous sodium sulfate, filtered, and concentrated to obtain compound 52-2 as an oil. This intermediate was directly used in the next step without separation.

Intermediate 52-2 obtained from the previous step was dissolved in acetic acid (6 mL) and ethanol (20 mL), and the solution was heated to 60 °C and allowed to react overnight. The starting materials were completely reacted as monitored by TLC. Saturated sodium bicarbonate was added to the reaction solution until it became alkaline, and then the mixture was extracted with ethyl acetate. The organic phases were combined, dried over anhydrous sodium sulfate, and concentrated. The residue was slurried with dichloromethane, and the slurry was filtered and dried at 50 °C to obtain compound 52 as an off-white solid (510 mg, three-step yield 22%). ¹H NMR (400 MHz, DMSO-*d*₆) δ 7.93 (s, 3H), 6.81 (s, 1H), 6.33 (d, *J =* 5.9 Hz, 1H), 5.40 (d, *J* = 5.9 Hz, 1H), 4.69 (t, *J* = 5.4 Hz, 1H), 4.32 (dd, *J =* 11.9, 2.6 Hz, 1H), 4.26 - 4.19 (m, 1H), 4.14 (dd, *J* = 11.9, 5.9 Hz, 1H), 3.97 - 3.88 (m, 1H), 2.02 (s, 3H). ESI-MS m/z = 335.2 [M+1]⁺.

### Example 11: Preparation of Compound 32

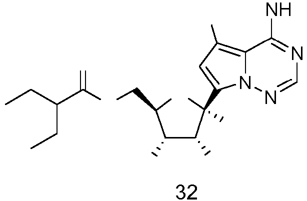

Referring to the synthesis method for compound 52 and using compound 4 (525 mg, 1.8 mmol) as a starting material, product 32 was obtained as a white solid (124 mg, three-step yield 47%). ¹H NMR (500 MHz, DMSO) δ 7.92 (s, 3H), 6.81 (s, 1H), 6.35 (d, *J =* 6.0 Hz, 1H), 5.39 (d, *J* = 5.8 Hz, 1H), 4.69 (t, *J* = 5.4 Hz, 1H), 4.29 (t, *J =* 7.5 Hz, 1H), 4.25 - 4.19 (m, 2H), 3.94 (q, *J* = 5.8 Hz, 1H), 2.20 - 2.13 (m, 1H), 1.50 - 1.41 (m, 4H), 0.78 (q, *J* = 7.3 Hz, 6H). ESI-MS m/z = 391.3 [M+1]⁺.

### Example 12: Preparation of Compound 53

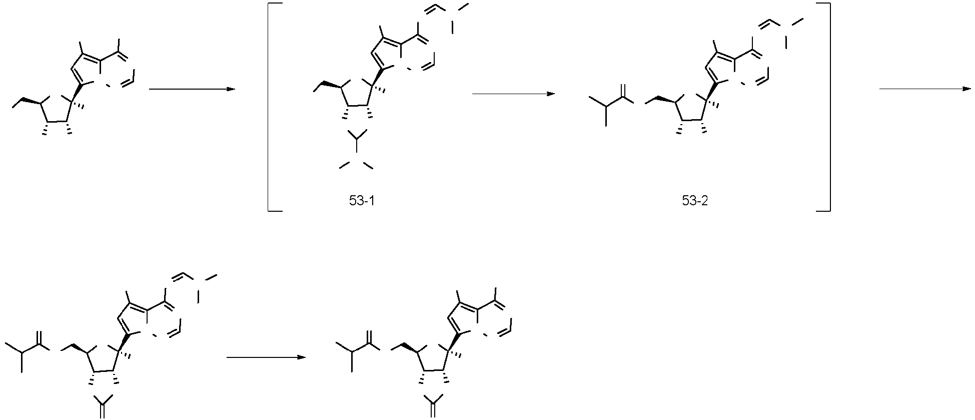

At room temperature, compound 4 (1.01 g, 3.46 mmol) was added to pyridine (40 mL), then *N,N-*dimethylformamide dimethyl acetal (1.66 g, 13.95 mmol) was added, and the mixture was stirred. After about 16 h, the reaction was completed, and the mixture was concentrated to dryness by rotary evaporation to remove the solvent to obtain compound 53-1 as an oil. This intermediate was directly used in the next step without separation.

At room temperature, intermediate 53-1 obtained from the previous step was added to pyridine (40 mL), and then 4-dimethylaminopyridine (48 mg, 0.39 mmol) was added, followed by the addition of isobutyric anhydride (826 mg, 5.23 mmol). After the mixture was stirred for 5 h, methanol was added. The resulting mixture was stirred for 30 min and then concentrated to dryness by rotary evaporation to remove the solvent to obtain compound 53-2 as an oil. This intermediate was directly used in the next step without separation.

Under an ice bath, intermediate 53-2 obtained from the previous step was added to tetrahydrofuran (30 mL), followed by the addition of carbonyldiimidazole (2.07 g, 12.7 mmol). The mixture was stirred, and the ice bath was removed. After about 12 h, the reaction was completed. The reaction solution was concentrated to dryness by rotary evaporation to obtain compound 53-3 as an oil. This intermediate was directly used in the next step without separation.

At 50 °C, intermediate 53-3 obtained from the previous step was added to an 80% glacial acetic acid-ethanol solution (50 mL), and the mixture was stirred. After about 2 h, the reaction was completed. The reaction solution was concentrated, and a saturated aqueous sodium bicarbonate solution was added. The mixture was extracted with ethyl acetate, and a saturated sodium chloride solution was added, followed by the addition of anhydrous sodium sulfate. The resulting mixture was filtered under vacuum and concentrated to dryness by rotary evaporation, and the residue was purified by silica gel column chromatography to obtain compound 53 as a white solid (583 mg, four-step yield 43%). ¹H NMR (500 MHz, DMSO-*d*₆) δ 8.11 (s, 1H), 8.03 (s, 1H), 7.98 (s, 1H), 6.90 (s, 1H), 5.99 (d, *J* = 7.7 Hz, 1H), 5.49 (dd, *J =* 7.6, 3.7 Hz, 1H), 4.82 (dt, *J =* 5.2, 3.8 Hz, 1H), 4.33 (dd, *J* = 12.3, 3.9 Hz, 1H), 4.22 (dd, *J* = 12.3, 5.2 Hz, 1H), 2.43 (m, *J* = 7.0 Hz, 1H), 1.01 (d, *J* = 7.0 Hz, 3H), 0.98 (d, *J* = 7.0 Hz, 3H). ESI-MS m/z = 389.4 [M+1]⁺.

### Example 13: Assay for Inhibitory Activity of Compounds of the Present Disclosure Against Virus:

CRFK cells in a good growth state were seeded into a 96-well assay plate. When the cells grew to 80%-90% confluence, feline coronavirus (FIPV) (0.1 MOI/well) and varying concentrations of the test compounds were added to the assay plate. Meanwhile, a cell control (cells, no compound treatment or viral infection) and a virus control (cells infected with virus, no compound treatment) were set. The final concentration of DMSO in the culture solution was 1%. After incubation at 37 °C for 72 h, the cells were treated with a CellTiter-Glo luminescent cell viability assay reagent (Promega, Madison, WI, USA). The half-maximal effective concentration (EC₅₀) values of the compounds for virus inhibition were calculated using the GraphPad prism 7 software.

**Table 1: In-vitro inhibitory activity of the compounds of the present disclosure against feline infectious peritonitis virus (FIPV)**

| Compound | 6 | 25 | 4 | 11 | 20 | 27 | 29 | 32 | 40 | 42 | 52 | 53 | GS-441524 |
|---|---|---|---|---|---|---|---|---|---|---|---|---|---|
| EC₅₀(µM) | 0.65 | 2.75 | 2.34 | 0.69 | 0.69 | 1.59 | 1.10 | 43.75 | 1.05 | 1.63 | 1.30 | 0.66 | 2.29 |

As can be seen from Table 1, the compounds of the present disclosure, especially compounds 6, 11, 20 and 53, all could inhibit the replication of feline infectious peritonitis virus (FIPV) *in vitro.*

### Example 14: Pharmacokinetic Evaluation in Rats

Male SD rats were fasted before the experiment (except for the intravenous group, which was not fasted), with free access to water, and were fed uniformly 2 h after administration. Compound 4 was administered by intravenous injection at a dose of 2.0 mg/kg (n = 3) and administered intragastrically at a dose of 10.0 mg/kg (n = 3); compound 53 was administered by intravenous injection at a dose of 5.0 mg/kg (n = 3) and administered intragastrically at a dose of 20.0 mg/kg (n = 3); the administration vehicle was 5% DMSO + 5% ethanol + 40% PEG300 + 50% saline. After the administration, 0.2-mL blood samples were collected via the jugular vein at different time points. Each blood sample was then placed into a heparin sodium anticoagulation tube, gently mixed uniformly, and centrifuged at 2000 g for 10 min. Plasma was isolated and frozen in a refrigerator at -70 °C for later analysis. The concentration of nucleoside (compound 4) in the plasma was determined by the LC-MS-MS method, and pharmacokinetic parameters were calculated. The results are shown in Table 2.

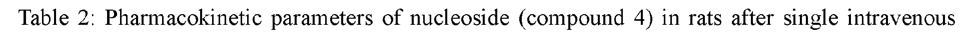

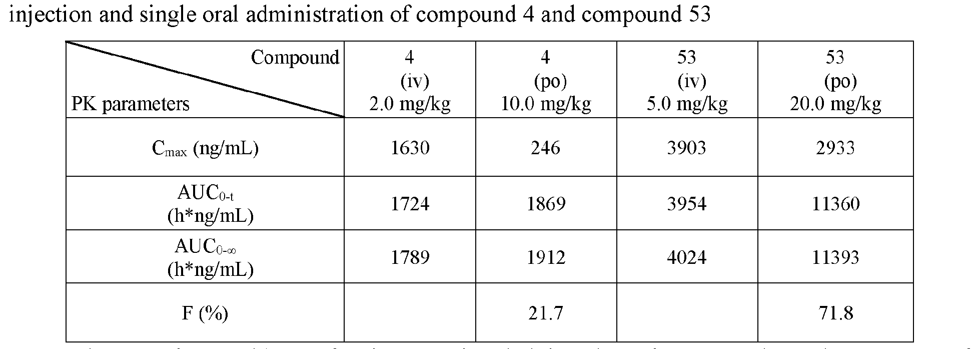

As can be seen from Table 2, after intragastric administration of compound 53, the exposure of nucleoside metabolite in the rats was high, with an oral bioavailability of 71.8%, which was significantly higher than the oral bioavailability of compound 4.

### Example 15: Pharmacokinetic Evaluation in Cats

Six male domestic cats were divided into 2 groups and fasted overnight before administration. The domestic cats were anesthetized by inhalation using isoflurane at a rate of 5 VOL%/L/min. After the domestic cats entered anesthesia stage III, the fur on the left hind leg of each animal was shaved, and an indwelling needle (for blood sampling) was inserted into the saphenous vein of the hind limb. Compound 6 and compound 27 were administered intragastrically at 10 mg/kg and 16 mg/kg, respectively, to 3 animals each. The vehicle for the compounds was 5% ethanol + 30% propylene glycol + 45% PEG 400 + 20% water. After administration, the domestic cats were placed in a prone position and secured to a surgical table to facilitate the subsequent blood collection. After intragastric administration, venous blood samples of 0.5-1 mL were collected at 15 min, 30 min, 1 h, 2 h, 4 h, 6 h, 8 h, and 24 h. A 200-µL whole blood sample was taken and centrifuged at 4 °C and 3000 rpm for 10 min. Then, 50 µL of the supernatant was taken, and 150 µL of methanol was added. The mixture was vortexed, centrifuged at 14800 rpm and 4 °C for 10 min, and then stored at -80 °C. The concentration of nucleoside metabolite (GS-441524) in the plasma was determined by the LC-MS-MS method, and pharmacokinetic parameters were calculated. The results are shown in Table 3.

**Table 3: Pharmacokinetic parameters of nucleoside (GS-441524) in rats after single oral administration of compound 6 and compound 27**

| | Animal No. | t_{1/2} | Tₘₐₓ | Cₘₐₓ | AUC₀₋ₜ |
|---|---|---|---|---|---|
| | | (h) | (h) | (ng/mL) | (ng·h/mL) |
| Compound 6 | 1 | 9.1 | 0.25 | 1700 | 9914 |
| | 2 | / | 0.50 | 2340 | 40603 |
| | 3 | / | 0.50 | 955 | 15093 |
| Compound 27 | 4 | 6.8 | 4.0 | 4290 | 36579 |
| | 5 | / | 4.0 | 1130 | 13263 |
| | 6 | 6.7 | 6.0 | 7180 | 78725 |

As can be seen from Table 3, after intragastric administration of compound 6 at 10 mg/kg, the Cₘₐₓ of the nucleoside metabolite in the animal plasma was 955-2340 ng/mL (3.28-8.04 µM); after intragastric administration of compound 27 at 16 mg/kg, the Cₘₐₓ of the nucleoside metabolite in the animal plasma was 1130-7180 ng/mL (3.88-24.67 µM); although there were significant individual differences among the animals, the Cₘₐₓ values of the nucleoside metabolite in the plasma were all significantly higher than the EC₅₀ value of GS-441524 against feline infectious peritonitis virus (FIPV).

It should be understood that the above examples are exemplary and are not intended to encompass all possible embodiments encompassed by the claims. Various modifications and changes may be made on the basis of the above examples without departing from the scope of the present disclosure. Likewise, the various features of the above examples may be arbitrarily combined to form additional examples of the present disclosure that may not be explicitly described. Therefore, the above examples only represent several embodiments of the present disclosure, and are not intended to limit the protection scope of the present disclosure.

## Claims

1. A compound represented by formula **I,** or a stereoisomer, a solvate, a hydrate, a prodrug, a stable isotopic derivative and a pharmaceutically acceptable salt thereof for use in the preparation of an inhibitor for inhibiting replication of feline coronavirus or calicivirus;
and/or a compound represented by formula I, or a stereoisomer, a solvate, a hydrate, a prodrug, a stable isotopic derivative and a pharmaceutically acceptable salt thereof for use in the preparation of a medicament for treating and/or preventing and alleviating a related disease caused by feline coronavirus or calicivirus infection: 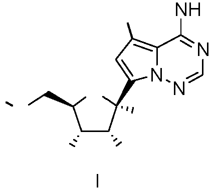
wherein
R₁ is selected from hydrogen, and substituted or unsubstituted C₁₋₂₀ alkanoyl and C₃₋₁₀ cycloalkylformyl, wherein the substitution is a substitution with one or more Q₁;
R₂ is selected from hydrogen, and substituted or unsubstituted C₁₋₂₀ alkanoyl and C₃₋₁₀ cycloalkylformyl, wherein the substitution is a substitution with one or more Q₂;
or R₁ and R₂ are connected with each other to form 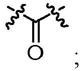 R₃ is selected from hydrogen, and substituted or unsubstituted C₁₋₂₀ alkanoyl and C₃₋₁₀ cycloalkylformyl, wherein the substitution is a substitution with one or more Q₃;
Q₁, Q₂, and Q₃ are each independently selected from hydrogen, cyano, amino, hydroxy, and halogen; R₄ is selected from hydrogen, deuterium, cyano, amino, hydroxy, and halogen.

2. The use according to claim 1, wherein R₁ in formula I is selected from hydrogen, and substituted or unsubstituted C₁₋₁₈ alkanoyl and C₃₋₇ cycloalkylformyl, preferably hydrogen, and substituted or unsubstituted C₁₋₁₈ alkanoyl and C₃₋₆ cycloalkylformyl, and more preferably hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl; Q₁ is selected from hydrogen, amino, hydroxy, and halogen, preferably hydrogen, amino, and halogen, and most preferably hydrogen and amino.

3. The use according to claim 1 or 2, wherein R₂ in formula I is selected from hydrogen, and substituted or unsubstituted C₁₋₁₈ alkanoyl and C₃₋₇ cycloalkylformyl, preferably hydrogen, and substituted or unsubstituted C₁₋₁₈ alkanoyl and C₃₋₆ cycloalkylformyl, and more preferably hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl; Q₂ is selected from hydrogen, amino, hydroxy, and halogen, preferably hydrogen, amino, and halogen, and most preferably hydrogen and amino.

4. The use according to any one of claims 1-3, wherein R₁ and R₂ in formula I are not simultaneously hydrogen.

5. The use according to any one of claims 1-4, wherein R₁ and R₂ in formula I are connected with each other to form

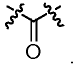

6. The use according to any one of claims 1-5, wherein R₃ in formula I is selected from hydrogen, and substituted or unsubstituted C₁₋₁₈ alkanoyl and C₃₋₇ cycloalkylformyl, preferably hydrogen, and substituted or unsubstituted C₁₋₁₈ alkanoyl and C₃₋₆ cycloalkylformyl, and more preferably hydrogen, formyl, acetyl, propionyl, butyryl, isobutyryl, valeryl, isovaleryl, pivaloyl, hexanoyl, 2-ethylbutyryl, 3,3-dimethylbutyryl, heptanoyl, octanoyl, 2-propylvaleryl, nonanoyl, decanoyl, C₁₄ alkanoyl, C₁₆ alkanoyl, C₁₈ alkanoyl, cyclopropylformyl, cyclobutylformyl, cyclopentylformyl, and cyclohexylformyl; Q₃ is selected from hydrogen, amino, hydroxy, and halogen, preferably hydrogen, amino, and halogen, and most preferably hydrogen and amino.

7. The use according to any one of claims 1-6, wherein R₄ in formula I is selected from hydrogen, deuterium, and halogen, preferably hydrogen, deuterium, fluorine, chlorine, and iodine.

8. The use according to any one of claims 1-7, wherein the compound represented by formula I is selected from any one of the following:

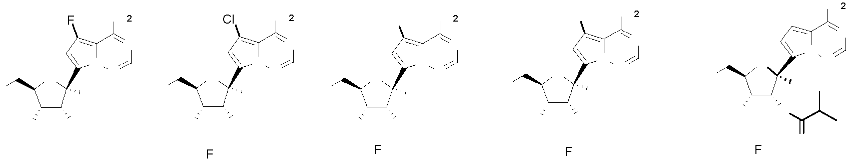

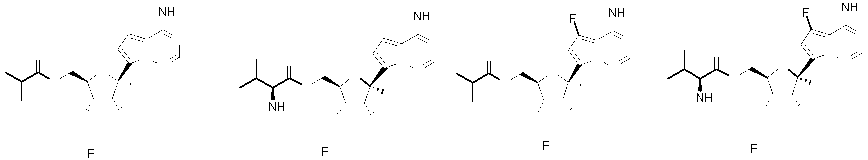

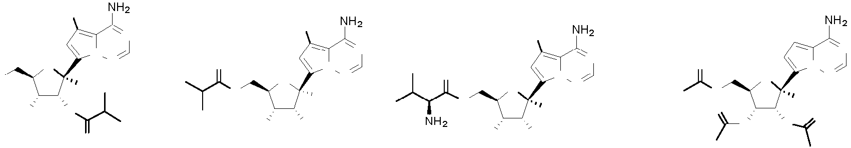

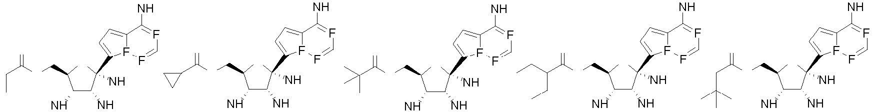

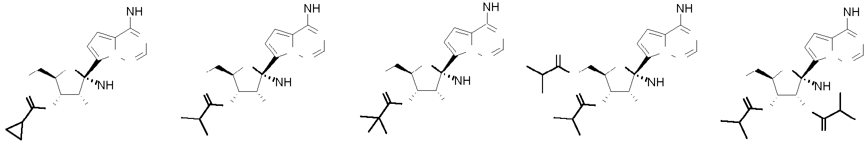

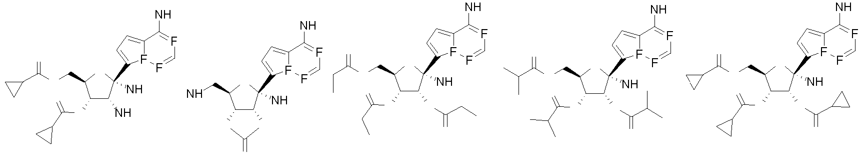

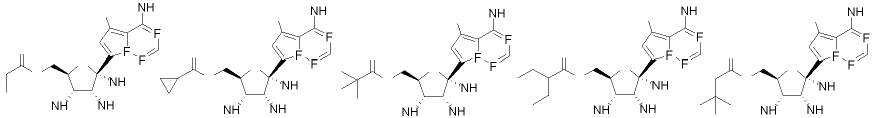

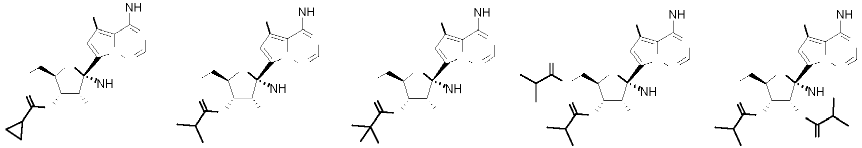

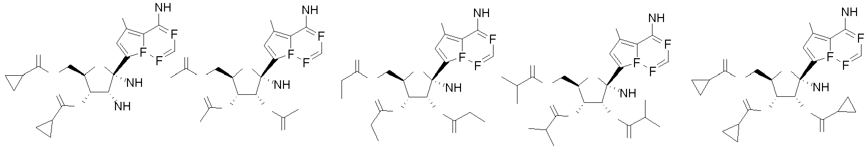

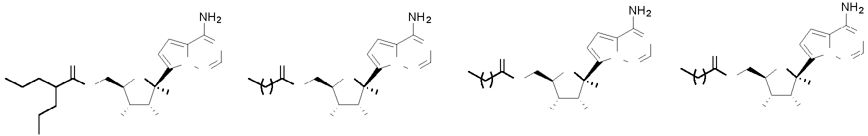

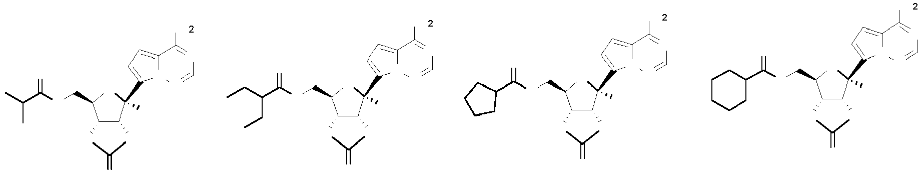

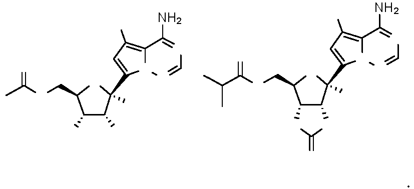

9. A composition comprising the compound represented by formula I, or the stereoisomer, the solvate, the hydrate, the prodrug, the stable isotopic derivative or the pharmaceutically acceptable salt thereof according to any one of claims 1-8 for use in the preparation of an inhibitor for inhibiting replication of feline coronavirus or calicivirus;
and/or a composition comprising the compound represented by formula I, or the stereoisomer, the solvate, the hydrate, the prodrug, the stable isotopic derivative and the pharmaceutically acceptable salt thereof according to any one of claims 1-8 for use in the preparation of a medicament for treating and/or preventing and alleviating a related disease caused by feline coronavirus or calicivirus infection.

10. The use according to any one of claims 1-9, wherein the related disease caused by feline coronavirus or calicivirus infection is selected from feline infectious peritonitis and feline stomatitis.
